# FASCICULE DE BREVET EUROPEEN

(11) **EP 0 681 428 B1**
(45) Date de publication et mention de la délivrance du brevet: **22.10.1997**
(21) Numéro de dépôt: 94904694.0
(22) Date de dépôt: 20.01.1994
(51) Int. Cl.: A01H 4/00

(54) **PROCEDE DE REGENERATION DU COCOTIER A PARTIR D'EXPLANTS**
VERFAHREN ZUR REGENERATION VON KOKOSPFLANZEN AUS EXPLANTATEN
PROCESS FOR REGENERATING THE COCONUT PALM FROM EXPLANTS

(30) Priorité: 29.01.1993 FR 9300986
(43) Date de publication de la demande: 15.11.1995
(73) Titulaire: INSTITUT FRANCAIS DE RECHERCHE SCIENTIFIQUE POUR LE DEVELOPPEMENT EN COOPERATION (ORSTOM), 75480 Paris Cédex 10 (FR); CENTRE DE COOPERATION INTERNATIONALE EN RECHERCHE AGRONOMIQUE POUR LE DEVELOPPEMENT (C.I.R.A.D.), 75116 Paris (FR)
(72) Inventeur: BUFFARD-MOREL, Jacqueline, F-34000 Montpellier (FR); PANNETIER, Catherine, F-92130 Issy-les-Moulineaux (FR); VERDEIL, Jean-Luc, F-34090 Montpellier (FR)
(74) Mandataire: Phélip, Bruno
(86) Numéro de dépôt international: FR9400067
(87) Numéro de publication internationale: WO9416551

(56) Documents cités:
- GB-A- 2 210 058
- CANADIAN JOURNAL OF BOTANY vol. 70, no. 4 , 1992 pages 735 - 741 J.BUFFARD-MOREL ET AL 'Embryogénèse somatique du coctier (Cocos nucifera L9 à partir d'explants foliaires : étude histologique' cité dans la demande
- OLEAGINEUX vol. 47, no. 7 , 1992 pages 465 - 469 J.L.VERDEIL ET AL 'Embryogénèse somatique du cocotier (Cocos nucifera L.) Obtention de plusieurs clones de vitroplants' cité dans la demande
- COCOA AND COCONUTS: PROGRESS AND OUTLOOK; PROCEEDINGS INT.CONF.COCOA AND COCONUTS KUALA LUMPUR 1984 1986 , KUALA LUMPUR pages 771 - 779 R.L.BRANTON & J.BLAKE 'Clonal propagation of coconut palm' cité dans la demande
- Y.P.S.BAJAJ -ED- 'Biotechnology in agriculture and forestry, Vol.10 Legumes and oilseed crops I' 1990 , SPRINGER VERLAG , BERLIN cité dans la demande Chapter IV.10, pages 538-554, J.BLAKE: "Coconut (Cocos nucifera L.) : Micropropagation". voir page 542, ligne 12 - page 552, ligne 14
- COCOA AND COCONUTS: PROGRESS AND OUTLOOK; PROCEEDINGS INT.CONF.COCOA AND COCONUTS KUALA LUMPUR 1984 1986 , KUALA LUMPUR pages 781 - 786 B.G.SMITH 'Tissue culture of Cocos nucifera - biochemical changes preceeding embryogenesis' cité dans la demande
- TISSUE CULTURE OF ECONOMICALLY INPORTANT PLANTS, PROCEEDINGS INTERNATIONAL SYMPOSIUM SINGAPORE 1981 1981, SINGAPORE, pages 145 - 148 J.BLAKE & C.J.EEUWENS 'Culture of coconut palm tissues with a view to vegatative propagation'
- Y.P.S.BAJAJ -ED- 'Biotechnology in agriculture and forestry, Vol.1 Tress I' 1986 , SPRINGER VERLAG , BERLIN cité dans la demande Chapter 13, pages 430-450, C.PANNETIER & J.BUFFARD-MOREL: " Coconut palm (Cocos nucifera L.)" voir page 438, ligne 34 - page 449, ligne 32
- W.R.SHARP ET AL -EDS- 'Handbook of plant cell culture, Vol.2. Crop species' 1984 , MACMILLAN PUBLISHING COMPANY , NEW YORK Chapter 18, pages 505-545, B.TISSERAT : "Date palm."
- ANNALS OF BOTANY vol. 52 , 1983 R.L.BRANTON & J.BLAKE 'Development of organized structures in callus derived from explants of Cocos nucifera L.'
- F.A.HAMMERSCHLAG & R.E.LITZ 'Biotechnology of perennial fruit crops' 1992 , C.A.B. INTERNATIONAL , WALLINGFORD UK Chapter 16, pages 383-400, A.K.BENBADIS :"Coconut and date palm"

## Description

La présente invention a pour objet un procédé de régénération du cocotier à partir d'explants .

Le cocotier a fait l'objet depuis les vingt dernières années d'un effort de sélection par les méthodes classiques qui ont conduit à l'obtention d'hybrides performants, présentant des rendements deux à trois fois supérieurs à ceux obtenus avec les variétés locales non sélectionnées.

La sélection classique se heurte cependant à différentes contraintes liées aux caractères morphologiques et à la biologie de cette plante :
- une allogamie fréquente responsable de la grande variabilité au sein des descendances et de l'hétérogénéité des plantations;
- des cycles de sélection très longs ( 12 à 16 ans);
- des coefficients de multiplication faibles , le cocotier étant uniquement propagé par graines et le nombre de noix par arbre restant peu élevé,
- une multiplication végétative inexistante dans les conditions naturelles, le cocotier ne possédant qu'un seul bourgeon végétatif en position terminale et ne formant pas de rejets.

La mise au point d'une méthode de multiplication végétative permettrait de contourner ces difficultés et d'obtenir un matériel homogène par clonage d'individus hautement performants, ce qui conduirait à une amélioration sensible de la productivité et de l'homogénéité des plantations.

Meunier et al.( Recent advances in genetic improvement of coconut yield. In : Pushprajah E., Chew Poh Soon (eds) Cocoa and Coconuts progress and outlook. Kuala Lumpur : Inc. Soc. Plant, pp 719-732 ( 1989)) , estiment que le clonage des meilleurs individus au sein d'une descendance hybride PB 121 devrait permettre théoriquement une augmentation de 24 % des rendements en coprah.

Les tentatives de régénération du cocotier ont abouti à la connaissance des demandeurs à l'obtention de quelques vitroplants mais n'ont pas conduit à la mise en oeuvre d'un procédé reproductible de régénération in vitro à partir d'explants .

Blake et Eeuwens ( Proc. Costed Symposium on Tissue Culture of Economically Important Plants , Singapore, 1981 Ed. A.N. Rao) décrivent les premières tentatives de multiplication in vitro du cocotier . Les auteurs mentionnent l'obtention de plantules à partir de fragments d'inflorescences mais les plants obtenus n'ont pu être enracinés.

Un compte-rendu de Branton et Blake ( 1984, International Conference on Cocoa and Coconuts ) mentionne l'obtention de deux vitroplants mais le procédé utilisé par ces auteurs conduit selon eux au développement, dans environ 9O% des cas, de structures embryoïdes anormales .

Buffard-Morel et al. ( Compte-Rendu du 8ème Congrès International sur les Biotechnologies, Paris, 1988) décrivent l'obtention d'une plantule à partir d'explants foliaires. Le milieu de régénération utilisé n'est pas indiqué.

Une revue parue ultérieurement ( Blake, Coconut Micropropagation , in Biotechnology in Agriculture and Forestry, Vol.10, Legumes and oil seeds crops ed. Y.P.S. Bajaj, pages 538-544, 1989) mentionne que des plantes ont parfois été obtenues par différentes équipes mais sans qu'il soit réellement possible de reproduire les conditions d'obtention et d'obtenir de grandes quantités de plantes .

L'article conclut que les résultats peu encourageants enregistrés dans la régénération du cocotier peuvent être expliqués par le fait que l'on s'attendait à ce que les tissus de cocotiers se comportent comme ceux du palmier à huile.

L'article le plus récent mentionnant l'obtention de vitroplants est un article de Verdeil et al. (Oléagineux , 1992 ,Vol. 47, n°7, pages 465-469 ).

Les auteurs mentionnent l'obtention d'une vingtaine de vitroplants . Selon ce procédé des cals sont obtenus sur un milieu additionné de 3O à 8O mg/l d'acide 2,4-dichlorophénoxyacétique ( 2,4-D) en présence de charbon actif (2 ou 3 g/l). Ces cals sont obtenus sur des explants soit foliaires soit inflorescenciels . Ils sont isolés de l'explant à partir du 6ème mois de culture et sont placés sur un milieu enrichi en 2,4-D par rapport à la concentration initiale, afin d'induire une embryogenèse. Les cals évoluent selon deux voies d'embryogenèse décrites par des études histologiques dont l'une conduit plus spécifiquement à la formation de pro-embryons, qui possèdent les caractéristiques des premiers stades de l'embryon zygotique.

La maturation de ces pro-embryons en embryons est obtenue en réduisant progressivement la concentration en 2,4-D utilisée lors de l'étape précédente . Le développement des pousses feuillées est réalisé sur un milieu dépourvu d'hormone et l'émission de racines , ou rhizogénèse , est induite par un traitement auxinique à base d'acide naphtalène acétique (ANA). Néanmoins , cette rhizogénèse peut parfois s'observer spontanément, c'est-à-dire sans traitement hormonal.

Enfin, l'article de BUFFARD-MOREL et al. (Journal Canadien de Botanique , 1992,7O(4): 735-741 ) décrit la formation d'une pousse feuillée sur un massif d'embryons , son isolement du massif et son enracinement. Ce vitroplant poursuit actuellement sa croissance en champ depuis 1989.

Cette plante a été régénérée à partir de cals obtenus sur un milieu gélosé contenant du saccharose , du charbon activé à raison de 2 à 3 g/l , ainsi que du 2,4-D à des concentrations variant de 3O à 6O mg/l.

L'induction de l'embryogenèse sur ces cals est effectuée sur des milieux appauvris progressivement en 2,4-D. Selon les auteurs les concentrations sont abaissées de O,22 10⁻⁴ à O,45 10⁻⁴ moles/l tous les quatres mois.

Aucune information n'est donnée concernant la maturation des embryons .

Il ressort de l'analyse de l'état de la technique ci-dessus qu'aucun procédé de régénération de cocotiers à partir d'explants , permettant l'obtention de plantes ne présentant pas d'anormalités, dans des conditions industrielles , n'a été décrit .

Les demandeurs se sont donc attachés à la mise au point d'un procédé permettant d'obtenir la multiplication d'un individu adulte donné de cocotier sans le léser, et notamment sans modifier ses capacités de production ou de reproduction .

Un autre objectif des demandeurs est un procédé ne provoquant pas de variabilité dans les plants qui en sont issus. Enfin, un tel procédé doit permettre l'obtention avec un rendement important d'un grand nombre de plants à partir d'un clone déterminé , dans des conditions de production industrielles .

Les demandeurs se sont heurtés au fait que les méthodes de régénération mises au point pour d'autres plantes tel que le palmier à huile, n'étaient pas transposables à la régénération du cocotier .

Ils ont montré de manière surprenante que l'on pouvait régénérer à partir d'explants obtenus sans léser le donneur , des plantes ayant des caractéristiques morphologiques normales par maturation des embryoïdes en embryons sur un milieu ne comprenant pas de charbon actif et d'auxine mais comprenant au moins une cytokinine.

La présente invention a donc pour objet un procédé de régénération du cocotier à partir d'explants comprenant les étapes de :
- induction de la formation de cals à partir d'explants,
- induction de l'embryogenèse sur les cals ,
- obtention de structures embryonnaires
- maturation des embryons,
- caulogenèse , et
- rhizogénèse.
caractérisé en ce que la maturation des embryons , est effectuée par repiquage de ces structures sur un milieu dépourvu de charbon actif et d'auxine , et comprenant au moins une cytokinine.

Avantageusement, la cytokinine est la 6-benzylaminopurine, la zéatine ( forme libre, riboside ou ribotide), l'isopentényl-adénine ( forme libre, riboside ou ribotide) , la kinétine ou la N,N₁-diphénylurée mais elle peut être toute autre cytokinine naturelle ou de synthèse, seule ou en combinaison .

La concentration en cytokinine peut être comprise entre O,1 et 10 mg/l de milieu , et préférentiellement entre 1 et 5 mg/l. Encore préférentiellement elle sera de l'ordre de 2,25 mg/l de milieu .

Avantageusement , l'induction des cals est effectuée sur un milieu gélosé comprenant une auxine et du charbon actif. Cette auxine est préférentiellement de l'acide 2,4-dichlorophénoxyacétique .

L'embryogenèse peut être induite sur les cals par augmentation de la quantité d'auxine dans le milieu gélosé sur lequel ils sont repiqués .

Les structures embryonnaires sont avantageusement obtenues à partir des structures embryogènes par réduction progressive de la quantité d'auxine dans le milieu.

La caulogénèse est avantageusement induite par repiquage des embryons obtenus après maturation , sur un milieu gélosé comprenant du charbon actif et dépourvu de facteur de croissance.

La rhizogénèse si elle n'est pas spontanée , peut être induite par transfert des embryons présentant une pousse feuillée sur un milieu contenant de l'acide naphtalène acétique ( ANA), ou de l'ANA et du charbon actif .

Avantageusement, les explants utilisés dans le procédé et sur lesquels on induit des cals sont des fragments de tissus foliaires ou des fragments d'inflorescence .

Les résultats obtenus dans l'étape de maturation des embryons sur un milieu dépourvu de charbon actif et contenant des facteurs de croissance, sont surprenants. L'homme du métier pour la reproduction in vitro du cocotier ayant plutôt tendance à adjoindre systématiquement du charbon actif dans des milieux contenant des facteurs de croissance .

Les techniques de manipulation des tissus végétaux in vitro ainsi que les techniques générales de régénération des plantes à partir d'explants , pouvant être utilisées dans le cadre de la présente invention sont celles décrites en particulier dans George E.F. et Sherrington P.D. (1984) Plant Propagation by Tissue Culture ,Exegetics Limited, Eversley, Basingstoke - Hants - RG 27 O9 Y, ENGLAND, Printed by Easter Press, Reading, Berks .

On se référera donc pour la mise en oeuvre du procédé objet de la présente demande à ce manuel général.

Ce procédé est avantageusement destiné à la régénération in vitro du cocotier , et en particulier des génotypes Nain jaune Malais, hybride Nain Jaune Malais x Grand Ouest Africain (PB 121), et hybride Grand Ouest Africain x Nain Rouge du Cameroun (PB111) créés par IRHO/CI, Grand-Ouest Africain, Nain rouge Cameroun et Nain vert .

La présente invention est illustrée sans pour autant être limitée par l'exemple suivant dans lequel :
La figure 1 représente un schéma général de mise en oeuvre du procédé objet de l'invention.
Les figures 2 et 3 sont des photographies respectivement d'un vitroplant selon la présente demande et d'un plant issu d'une noix obtenue par la technique classique de reproduction .

### EXEMPLE :

### Multiplication in vitro du cocotier à partir de fragments de feuilles et d'inflorescences .

### a) Formation de cals.

La callogenèse conduit à la formation de tissu inorganisé ou cal, à partir de fragments de feuilles ou d'inflorescences immatures.

### 1) collecte du matériel au champ.

Des feuilles immatures ( feuilles de rang F-6 et F-7, FO étant la première feuille déployée) et des inflorescences immatures , situées à l'aisselle des feuilles de rang F+2, F+3, F+4, sont collectées au champ .

Ces deux sources d'explants sont collectées sur des arbres adultes de génotype Nain Jaune Malais, hybride Nain Jaune Malais x Grand Ouest Africain (PB 121), hybride Grand Ouest Africain x Nain Rouge du Cameroun (hybride PB 111) qui sont des hybrides crées par le département oléagineux du CIRAD, sans léser le bourgeon terminal, ce qui permet la survie de la plante-mère.

### 2) Désinfection du matériel et mise en culture.

Le matériel est désinfecté superficiellement par une solution d'eau de javel du commerce diluée seize fois.

Les explants sont fragmentés sous hotte à flux laminaire, puis chaque fragment est placé dans une solution d'attente anti-oxydante afin d'éviter le brunissement des explants (3O g/l de saccharose, 100 mg/l d'ascorbate de sodium et 150mg/l d'acide citrique).

Les explants sont alors repiqués sur le milieu de callogenèse ayant la composition suivante:
- solution minérale de Eeuwens (Physiol. Plant, 36, 23-28, (1976)) pour les macro et les microéléments,
- vitamines de Morel et Wetmore ( 1951- Fern callus tissue culture. Amer. J. Bot. 38:141-143),
- ascorbate de sodium 100 mg/l
- saccharose 3O g/l,
- 2,4-D (Sigma) 44, 55 et 66 mg/l
- charbon actif neutralisé (Sigma) 2 g/l
- bacto agar (Sigma) 7,5 g/l
- eau désionisée Millipore, q.s.p. 1 l.

Le pH est ajusté à 4,5 avant adjonction de l'agar puis du charbon actif.

Les milieux sont distribués dans des tubes de 24 x 160 mm fermés par un capuchon en verre et autoclavés à 115°C pendant 3O minutes.

### 3) Stockage des explants mis en culture.

Les tubes de culture sont placés à l'obscurité dans une chambre de culture maintenue à 27°C ± 1°C et ils sont incubés pendant plusieurs mois jusqu'à l'apparition des cals (entre 3 et 10 mois suivant les génotypes ).

### b) Isolement des cals et induction de l'embryogenèse.

### 1) Matériel végétal utilisé.

Deux types de cals pouvant être distingués sur des critères morphologiques sont obtenus:
a) des cals nodulaires jaunâtres ,
b) des cals blancs de type granuleux.

L'embryogenèse est obtenue sur les deux types de cals.

Ce type de cal granuleux est obtenu sur explants inflorescenciels, directement à partir des bourgeons floraux durant l'étape de callogenèse. Sur explants foliaires ils apparaissent après l'isolement des cals primaires nodulaires et compacts.

### 2) Milieu d'embryogenèse et conditions d'obtention de structures embryogènes.

L'embryogénèse est obtenue par repiquage des cals sur deux milieux contenant l'un 8O mg/l de 2,4-D, 2 g/l de charbon, 4O g/l de saccharose et 4O mg/l de sulfate d'adénine (milieu M3), et l'autre 120 mg/l de 2,4-D, 3 g/l de charbon actif, 2O mg/l de sulfate d'adénine et 2O g/l de glucose (milieu G3). La solution minérale utilisée est celle de Murashige et Skoog. (Physiol. Plant, 15, 473-497, (1962)).

Le repiquage des cals sur le milieu d'embryogenèse se fait régulièrement tous les deux mois.

Les cultures sont maintenues à l'obscurité, à 27°C.

Dans ces conditions de culture, des structures embryogènes blanches et épidermisées apparaissent entre 2 et 2O mois après le premier repiquage sur au plus 1O% des cals isolés à partir des trois génotypes.

### c) Obtention des structures embryonnaires et maturation des embryons.

Les structures blanches et épidermisées sont repiquées sur des milieux progressivement appauvris en 2,4-D. Lorsque les structures embryogènes évoluent en structures embryonnaires, elles sont repiquées sur un milieu dépourvu de 2,4-D et de charbon actif et contenant une cytokinine: la BAP à raison de 2,25 mg/l (milieu CK3).

Le milieu de base est le suivant :
- macro éléments de Murashige et Skoog (MS)
- micro éléments de Murashige et Skoog modifiés par Nitsch (MSNC)( Nitsch (1969) Phytomorph. 19, 389-4O4)
- vitamines de Morel et Wetmore ( précédemment cités)
- saccharose 3O g/l.

Les structures embryonnaires obtenues sont repiquées tous les deux mois sur ce milieu jusqu'à émission de la première gaine foliaire.

### d) Isolement des embryons et caulogenèse.

Les embryons sont transférés, après émission de la première feuille en forme d'écaille sur le milieu suivant : solutions minérales MS et MSNC, hydrolysat de caséine 5OO mg/l, saccharose 3O g/l, bacto agar 7,5 g/l et charbon actif 2 g/l. Les embryons peuvent alors être transférés dans une salle lumineuse ( photopériode de 12 heures) . Ils sont repiqués tous les deux mois jusqu'à ce que les pousses feuillées atteignent 5 à 8 cm de hauteur.

### e) Enracinement des embryons ayant émis leur pousse feuillée.

Deux types de milieu peuvent être utilisés pour l'enracinement :
- soit un milieu gélosé renfermant le milieu de base standard, 6O g/l de saccharose, 5OO mg/l d'hydrolysat de caséine, 2O mg/l d'ANA, 2 g/l de charbon actif, bacto agar 7,5 g/l. Le pH de ce milieu est ajusté à 5,6,
- soit un milieu liquide renfermant le milieu standard, 2O g/l de saccharose et O,5 à 2 mg/l d'ANA.

Dans le premier cas (a) après apparition des premières racines ( 2 mois environ), le vitroplant est transféré sur un milieu de même composition, mais dépourvu d'ANA et contenant du charbon actif à 2 g/l.

Dans le deuxième cas (b) le vitroplant est transféré après 24 à 48 heures sur le même milieu dépourvu d'ANA mais renfermant 6O g/l de saccharose.

### f ) Caractéristiques des plantules obtenues.

Environ quarante vitroplants de différents génotypes ont été obtenues par ce procédé.

Aucun des vitroplants obtenus en tubes ne présente d'anomalies morphologiques.

En début d'émission des pousses feuillées, les vitroplants se distinguent des plants issus de noix obtenues par les techniques classiques de reproduction par les caractéristiques suivantes :
- le cotylédon des plants obtenus in vitro reste très petit et est généralement fusiforme, à la différence de celui de plants issus de la culture d'embryons zygotiques dans lesquels il devient très volumineux et occupe toute la cavité de la noix ,
- les plants obtenus in vitro sont de taille plus faible, à stades de croissance comparables, à ceux issus de noix d'un même génotype ,
- le port des plants obtenus in vitro est différent . Il est plus étalé , les feuilles sont plus ramassées et présentent des pétioles et des limbes plus courts que ceux de plants issus de noix à des stades de croissance comparables.

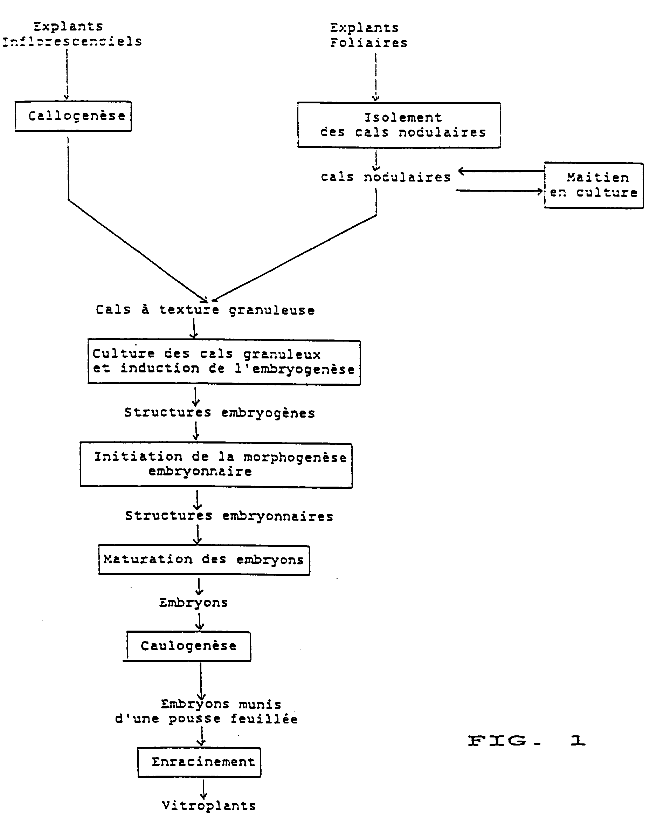

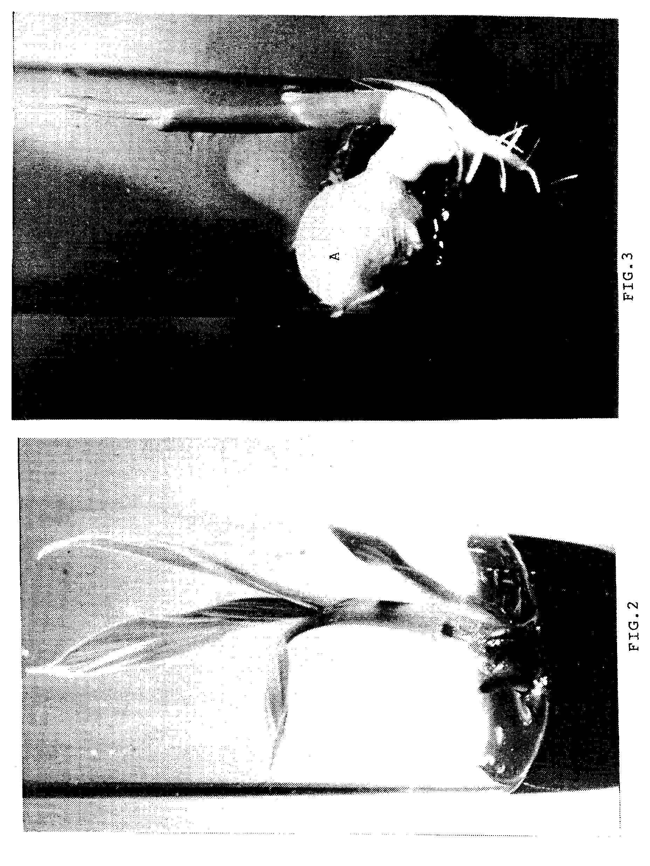

Ces différences ressortent clairement de la comparaison des photographies des figures 2 et 3 qui représentent respectivement un vitroplant selon l'invention et un plant obtenu classiquement par embryogenèse zygotique . Sur la figure 3, (A) indique le cotylédon.

Les comparaisons données ci-dessus s'entendent pour des plants à des stades de croissance comparables . Ces différences s'estompent progressivement avec le développement des plants.

Il s'agit de comparaisons statistiques représentant les morphologies moyennes des populations.

## Revendications

1. Procédé de régénération du cocotier à partir d'explants comprenant les étapes de :
- induction de la formation de cals à partir d'explants,
- induction de l'embryogenèse sur les cals ,
- obtention de structures embryonnaires,
- maturation des embryons,
- caulogenèse et,
- rhizogenèse.
caractérisé en ce que la maturation des embryons est effectuée par repiquage de ces structures sur un milieu dépourvu de charbon actif et d'auxine, et comprenant au moins une cytokinine.

2. Procédé selon la revendication 1, caractérisé en ce que la cytokinine est la 6-benzyl-aminopurine, la zéatine, l'isopentényl-adénine, la kinétine , ou la N, N1-diphénylurée.

3. Procédé selon l'une des revendications 1 à 2, caractérisé en ce que la concentration en cytokinine est comprise entre O,1 et 10 mg/l de milieu, préférentiellement entre 1 et 5 mg/l de milieu et encore plus préférentiellement est de l'ordre de 2,25 mg/l de milieu.

4. Procédé selon l'une des revendications 1 à 3, caractérisé en ce que les cals sont induits sur un milieu gélosé comprenant une auxine et du charbon actif.

5. Procédé selon la revendication 4, caractérisé en ce que l'auxine est l'acide 2,4-dichlorophénoxyacétique.

6. Procédé selon l'une des revendications 1 à 5, caractérisé en ce que l'embryogenèse est induite par augmentation de la quantité d'auxine dans le milieu gélosé.

7. Procédé selon l'une des revendications 1 à 6, caractérisé en ce que la caulogenèse est induite par repiquage des embryons sur un milieu gélosé comprenant du charbon actif et dépourvu de facteur de croissance .

8. Procédé selon l'une des revendications 1 à 7, caractérisé en ce que la rhizogenèse est induite par transfert des embryons présentant une pousse feuillée sur un milieu gélosé contenant du charbon actif et de l'acide naphtalène acétique .

9. Procédé selon l'une des revendications 1 à 7, caractérisé en ce que la rhizogenèse est induite par transfert sur un milieu liquide renfermant du saccharose et de l'acide naphtalène acétique.

10. Procédé selon l'une des revendications 1 à 9, caractérisé en ce que les explants sont des fragments de tissus foliaires ou d'inflorescences .

## Claims

1. Process for the regeneration of the coconut palm from explants comprising the stages of :
- induction of callus formation from explants,
- induction of embryogenesis on the calluses,-obtaining embryonic structures,
- maturation of the embryos,
- caulogenesis, and
- rhizogenesis.
characterized in that the maturation of the embryos is carried out by subculturing these structures on a medium containing neither activated carbon nor auxin, and containing at least a cytokinin.

2. Process according to claim 1, characterized in that the cytokinin is 6-benzylaminopurine, zeatin, isopentyl-adenine, kinetin, or N,N'-diphenylurea.

3. Process according to any one of claims 1 to 2, characterized in that the concentration of cytokinin is between 0.1 and 10 mg/l of medium, and preferably between 1 and 5 mg/l, and even more preferably of the order of 2.25 mg/l of medium.

4. Process according to any one of claims 1 to 3, characterized in that the calluses are induced on an agar-agar medium containing an auxin and activated carbon.

5. Process according to claim 4, characterized in that the auxin is 2,4-dichlorophenoxyacetic acid.

6. Process according to any one of claims 1 to 5, characterized in that the embryogenesis is induced by increasing the quantity of auxin in the agar-agar medium.

7. Process according to any one of claims 1 to 6, characterized in that the caulogenesis is induced by subculturing the embryos on an agar-agar medium containing activated carbon but no growth factor hormone.

8. Process according to any one of claims 1 to 7, characterized in that the rhizogenesis is induced by transferring those embryos showing a leaf shoot onto an agar-agar medium containing activated carbon and naphthaleneacetic acid.

9. Process according to any one of claims 1 to 7, characterized in that the rhizogenesis is induced by transfer into a liquid medium containing sucrose and naphthaleneacetic acid.

10. Process according to any one of claims 1 to 9, characterized in that the explants are fragments of foliar tissue or inflorescences.

## Patentansprüche

1. Verfahren zur Regeneration von Kokospalmen aus Explantaten, umfassend die Schritte der
- Induktion der Bildung von Kallusen aus Explantaten,
- Induktion der Embryogenese bei den Kallusen,
- Gewinnung von embryonalen Strukturen,
- Reifung der Embryonen,
- Sproßbildung und
- Wurzelbildung,
dadurch gekennzeichnet, daß die Reifung der Embryonen durch Verpflanzen dieser Strukturen auf ein Medium erfolgt, das frei von aktivem Kohlenstoff und von Auxin ist und das wenigstens ein Cytokinin umfaßt.

2. Verfahren gemäß Anspruch 1, dadurch gekennzeichnet, daß es sich bei dem Cytokinin um 6-Benzylaminopurin, Zeatin, Isopentenyladenin, Kinetin oder N,N'-Diphenylharnstoff handelt.

3. Verfahren gemäß einem der Ansprüche 1 bis 2, dadurch gekennzeichnet, daß die Konzentration des Cytokinins zwischen 0,1 und 10 mg/l des Mediums, vorzugsweise zwischen 1 und 5 mg/l des Mediums und noch mehr bevorzugt in der Größenordnung von 2,25 mg/l des Mediums liegt.

4. Verfahren gemäß einem der Ansprüche 1 bis 3, dadurch gekennzeichnet, daß die Kalluse auf einem Gelosemedium induziert werden, das ein Auxin und aktiven Kohlenstoff umfaßt.

5. Verfahren gemäß Anspruch 4, dadurch gekennzeichnet, daß es sich bei dem Auxin um 2,4-Dichlorphenoxyessigsäure handelt.

6. Verfahren gemäß einem der Ansprüche 1 bis 5, dadurch gekennzeichnet, daß die Embryogenese durch Erhöhung der Menge des Auxins in dem Gelosemedium induziert wird.

7. Verfahren gemäß einem der Ansprüche 1 bis 6, dadurch gekennzeichnet, daß die Sproßbildung durch Verpflanzen der Embryonen auf ein Gelosemedium induziert wird, das aktiven Kohlenstoff umfaßt und frei von Wachstumsfaktor ist.

8. Verfahren gemäß einem der Ansprüche 1 bis 7, dadurch gekennzeichnet, daß die Wurzelbildung durch Übertragen der Embryonen, die einen beblätterten Sproß aufweisen, auf ein Gelosemedium induziert wird, das aktiven Kohlenstoff und Naphthalinessigsäure enthält.

9. Verfahren gemäß einem der Ansprüche 1 bis 7, dadurch gekennzeichnet, daß die Wurzelbildung durch Übertragen auf ein flüssiges Medium induziert wird, das Saccharose und Naphthalinessigsäure enthält.

10. Verfahren gemäß einem der Ansprüche 1 bis 9, dadurch gekennzeichnet, daß es sich bei den Explantaten um Fragmente von Blattgeweben oder Blütenständen handelt.
